# EUROPEAN PATENT APPLICATION

(11) **EP 4 549 914 A1**
(43) Date of publication of application: **07.05.2025**
(21) Application number: 23000154.7
(22) Date of filing: 06.11.2023
(51) Int. Cl.: G01N 21/65, G01J 3/44, A61B 5/00, A61B 5/1459, A61B 5/145

(54) **IMPLANTABLE DEVICE, METABOLITE MONITORING SYSTEM AND METHOD OF MEASURING METABOLITES WITH COMMUNICATION LAYER**

(71) Applicant: Walletmed Limited, Dublin D07 P4AX (IE)
(72) Inventor: Paprota, Wojciech, 21-030 Motycz (PL); Miturski, Andrzej, 21-003 Jakubowice Koninskie (PL); Gasior, Michal, 05-270 Marki (PL); Buszynski, Jakub, 60-452 Poznan (PL)
(74) Representative: Korbela, Anna

(57) **Abstract**

Embodiments of the present disclosure may include an implantable device (100) for *in vivo* measurement of metabolites, including a laser diode (110) or an IR diode (120) or optical waveguide as an external (outside body) light source with a laser diode (110) or an IR diode (120) for Raman spectrometry unit (140). Embodiments may also include an optical filter (130) that filters the scattered light. Embodiments may also include a Raman spectroscopy unit (140) for detecting the metabolic profile of a subject. Embodiments may also include a Fabry-Pérot interferometer (150) with a spherical mirror, functioning as an etalon, for refining the detection of Raman scattering signals. Embodiments may also include a CMOS sensor (160) for capturing the refined Raman scattering signals. Embodiments may also include a piezo element (170a) or MEMS actuator (170b) for actuating the Fabry-Pérot interferometer (150). Embodiments may also include MCU (180) as a controller and a processing unit.

## Description

The subject of the invention is an implantable device for monitoring metabolites including, but not limited to, amino acids, lipids, carbohydrates, nucleic acids, vitamins, cofactors or metabolic intermediates, and a method for communicating the results of metabolite measurements. The developed implantable device uses Raman spectroscopy and Fabry-Perot interferometry with a spherical semi-permeable mirror in etanol to analyze multiple metabolites *in vivo* and provide real-time feedback. The developed metabolite monitoring system is/will be mainly used in medicine, veterinary medicine, laparoscopy, especially for analyzing the chemical compositions of substances including liquid, solid, bulk materials.

Various metabolite monitoring methods are known, for example the solution "Metabolite Management System" is known from the American invention application no. US2010256466A1 (priority date: 02.04.2009). Developed metabolite monitoring system is configured to deliver drugs and obtain metabolite samples via one common skin perforation and measure metabolite content by way of an optical measuring-cell having a primary flowpath of constant cross-sectional area. This solution requires taking a sample, which is analyzed. In this solution we have a typical cannula, it is invasive and requires frequent replacement and change of the implantation site, due to permanent perforation of the skin layers.

Whereas the solution "System, devices, and methods for real-time monitoring of cerebrospinal fluid for markers of progressive conditions" is known from another American invention no. US2012238836A1 (priority date 16-03-2011). Systems, devices, methods, and compositions are described for providing real-time monitoring of cerebrospinal fluid for markers of progressive conditions. A method for predicting an onset of a depressive disorder, comprising transcutaneously communicating a suicidal tendency status in response to an *in vivo* comparison of cerebrospinal fluid neuropeptide compositional information of a cerebrospinal fluid received within the one or more fluid-flow passageways of an indwelling implant to reference mental disorder filtering information.

The purpose of the developed invention is to create a reagent free (non-enzymatic, alloptical), non-invasive device and method (invasive action is only necessary at the stage of implantation of the device, however, its subsequent use is not associated with interference with the user's body). There is no need to replace the device for at least 10 years.

The aim of the developed solution is the creation of an innovative, long-term implantable device that enables continuous, real-time metabolite monitoring through a minimally invasive procedure. Utilizing advanced optical technologies, such as Raman spectroscopy combined with Fabry-Pérot interferometry, the device is designed to detect a wide spectrum of metabolites without the need for reagents, external sampling, or frequent maintenance. This ensures a safer, more convenient method for patients and animals, with decreased risk of infection or skin perforation compared to traditional methods. The system's communication layer facilitates seamless data transfer to external devices for immediate analysis, allowing for proactive health management and a more personalized approach to medical care.

The essence of the invention is an implantable device for *in vivo* measurement of metabolites which comprises,
- a laser diode or an IR (infrared) diode,
- an optical filter that filters the scattered light,
- a Raman spectroscopy unit for detecting the metabolic profile of a subject,
- a Fabry-Pérot interferometer with a spherical mirror, functioning as an etalon, for refining the detection of Raman scattering signals,
- a CMOS sensor for capturing the refined Raman scattering signals,
- a piezo element or MEMS actuator for actuating the Fabry-Pérot interferometer,
- MCU as a controller and a processing unit.

Preferably, the laser diode or the IR (infrared) diode is inside or outside as an optical waveguide ring with laser diode or IR diode as a light source.

Preferably, the implantable device includes the Fabry-Pérot interferometer with a flat and spherical semi-transparent mirrors and the piezo element or MEMS actuator to adjust spacing between semi- transparent mirrors, refining the detection of specific metabolite-related Raman scattering light wavelengths.

Preferably, the semi-transparent mirrors are created from optical fiber bundle or optical fiber plate or homogenous glass.

Preferably, the MCU as the data processing unit within the device analyzes interference fringes from Fabry-Pérot interferometer as the CMOS sensor generated electrical signal to determine metabolite identity and concentration, employing machine learning or pattern recognition techniques aligned with known Raman spectra.

Preferably, the implantable device encompasses a communication module, preferably with wireless capabilities, to transmit metabolite data to external devices for further processing, display, or storage.

Preferably, the implantable device is biocompatible for long-term implantation, supported by a power buffer unit, which can be recharged or sustained via energy harvested from external sources like inductive coupling.

The essence of the invention is also a method for *in vivo* measurement of metabolites using the above-mentioned implantable device, comprising the steps of:
a. generating light using laser diode or IR diode or optical waveguide ring with laser diode or IR diode,
b. generating Raman scattering signals from the subject's tissue using the Raman spectroscopy unit,
c. refining the detection of Raman scattering signals with the Fabry-Pérot interferometer and flat and the spherical semi-transparent mirrors,
d. capturing the refined Raman scattering signals using the CMOS sensor,
e. converting the captured Raman scattering signals into an electrical signal representative of the metabolite concentrations in the subject's tissue using the CMOS sensor,
f. controlling the spacing between flat and spherical semi-transparent mirrors in the Fabry-Pérot interferometer using the piezo element or MEMS actuator to fine-tune the detected Raman scattering signals,
g. processing the electrical signal to determine the concentration and identity of the metabolites in the subject's tissue using a MCU as a data processing unit,
h. transmitting the metabolite concentration and identity information to an external device for further analysis, display, or storage using a communication module and
i. powering the implantable device using a power buffer unit and supplying the power through inductive coupling of external device.

Preferably, the method includes steps for generating and refining Raman signals, converting them to an electrical representation, processing this data to discern metabolite details, and transmitting this information externally, all powered *via* the short-range wireless connectivity.

The essence of the invention is also a system for *in vivo* measurement of metabolites, comprising:
a. the implantable device,
b. an external device for receiving and processing the transmitted metabolite concentration and identity information,
c. a user interface for displaying the metabolite concentration and identity information to a user.

Preferably, the system receives updates to enhance performance, interface with other devices or health records, and generate alerts based on specific metabolite information parameters or thresholds.

Preferably, the implantable device of the system is placed within a subject to measure metabolite concentrations and identities *in vivo* using Raman scattering signals and this process includes refining signals with specific equipment and processing these signals to identify the metabolites.

Preferably, the system uses advanced techniques, like machine learning, refine the data analysis and users can access support channels for troubleshooting, and training is provided to ensure proper system usage.

Preferably, the system is used in diverse applications, including research and various clinical settings.

The essence of the invention is also a method for monitoring metabolite concentrations and identities in a subject's tissue using the above-mentioned system, comprising the steps of:
a. implanting the implantable device in the human or another animal body,
b. measuring metabolite concentrations and identities *in vivo* using the implantable device, including generating Raman scattering signals, refining the signals with the Fabry-Pérot interferometer and the flat and spherical semi-transparent mirrors, capturing the signals using the CMOS sensor, and processing the signals to determine metabolite concentrations and identities,
c. transmitting the metabolite concentration and identity information from the implantable device to the external device using wireless communication protocols,
d. processing and analyzing the received metabolite concentration and identity information on the external device, including comparison with reference data trending or statistical analysis,
e. displaying the metabolite concentration and identity information on the user interface and
f. generating alerts or notifications based on predetermined thresholds, patterns, or changes in the metabolite concentration and identity information using an alert module.

Preferably, the implantable device wirelessly transmits the data to an external device, where it is processed, stored for future reference, and analyzed and this includes comparison with other data and the potential for historical analysis, security and privacy mechanisms protect this data.

Preferably, the external device provides a customizable user interface to display the metabolite data, generating alerts based on specific criteria and users receive updates or configuration changes for the system, and there are options for synchronization and integration with other devices or health records.

Preferably, the implantable device is adapted its measurements based on various factors related to the subject, it offers feedback to the subject or healthcare providers, including potential recommendations based on the data.

Preferably, the advanced techniques, like machine learning, refine the data analysis, users access support channels for troubleshooting, and training is provided to ensure proper system usage, the system is used in diverse applications, including research and various clinical settings.

Embodiments may also include a Fabry-Pérot interferometer with a spherical mirror, functioning as an etalon, for refining the detection of Raman scattering signals. Embodiments may also include a CMOS sensor for capturing the refined Raman scattering signals. Embodiments may also include a piezo element for actuating the Fabry-Pérot interferometer. Embodiments may also include MCU as a controller and a processing unit.

Embodiments of the present disclosure may also include, the implantable device of claim 1. In some embodiments, the Raman spectroscopy unit may be configured to generate Raman scattering signals in response to incident light interacting with the metabolites in the subject's tissue.

Embodiments of the present disclosure may also include, the implantable device of claim 1. In some embodiments, the Fabry-Pérot interferometer with a spherical mirror may be configured to selectively filtering specific wavelengths of the Raman scattering signals corresponding to the metabolites of interest.

Embodiments of the present disclosure may also include, the implantable device of claim 1. In some embodiments, the CMOS sensor may be configured to convert the transmitted Raman scattering signals into an electrical signal representative of the metabolite concentrations in the subject's tissue.

Embodiments of the present disclosure may also include, the implantable device of claim 1. In some embodiments, the piezo element may be configured to control the spacing of the spherical mirror in the Fabry-Pérot interferometer, thereby allowing for fine-tuning of the detected Raman scattering signals.

Embodiments of the present disclosure may also include, the implantable device of claim 1, further including a data processing unit for analyzing the electrical signal generated by the CMOS sensor to determine the concentration and identity of the metabolites in the subject's tissue.

Embodiments of the present disclosure may also include the implantable device of claim 1. In some embodiments, the data processing unit may be configured to apply machine learning algorithms for pattern recognition techniques to analyze the electrical signal and correlate it with known Raman spectra of various metabolites.

Embodiments of the present disclosure may also include the implantable device of claim 1, further including a communication module for transmitting the metabolite concentration and identity information to an external device for further analysis, display, or storage.

Embodiments of the present disclosure may also include the implantable device of claim 1. In some embodiments, the communication module may be configured to use wireless communication protocols for data transmission to the external device.

Embodiments of the present disclosure may also include the implantable device of claim 1. In some embodiments, the device may be biocompatible and configured for long-term implantation in the subject's body (human or another animal).

Embodiments of the present disclosure may also include, the implantable device of claim 1, further including a power buffer unit. In some embodiments, the power supply unit may be configured to provide power to the Raman spectroscopy unit, the Fabry-Pérot interferometer, the CMOS sensor, the piezo element, and any additional components.

Embodiments of the present disclosure may also include, the implantable device of claim 1. In some embodiments, the power supply unit may be configured to harvest energy from external sources, such as through inductive coupling, to recharge the power buffer unit or maintain the device's power level.

Embodiments of the present disclosure may also include a method for *in vivo* measurement of metabolites using the implantable device of claim 1, including the steps of generating light using laser or IR diode or external optical waveguide ring with laser or IR diode that works as light source. Embodiments may also include generating Raman scattering signals from the subject's tissue using the Raman spectroscopy unit. Embodiments may also include refining the detection of Raman scattering signals with the Fabry-Pérot interferometer and the spherical mirror that works as a special etalon.

Embodiments may also include capturing the refined Raman scattering signals using the CMOS sensor. Embodiments may also include converting the captured Raman scattering signals into an electrical signal representative of the metabolite concentrations in the subject's tissue using the CMOS sensor. Embodiments may also include controlling the spacing of the spherical mirror in the Fabry-Pérot interferometer using the piezo element to fine-tune the detected Raman scattering signals.

Embodiments may also include processing the electrical signal to determine the concentration and identity of the metabolites in the subject's tissue using a data processing unit. Embodiments may also include transmitting the metabolite concentration and identity information to an external device for further analysis, display, or storage using a communication module. Embodiments may also include powering the implantable device using a power buffer unit and supplying the power through inductive coupling of external devices.

Embodiments of the present disclosure may also include the method of claim 15. In some embodiments, the data processing step may include applying machine learning algorithms for pattern recognition techniques to analyze the electrical signal and correlate it with known Raman spectra of various metabolites.

Embodiments of the present disclosure may also include the method of claim 15. In some embodiments, the transmission step may include using wireless communication protocols for data transmission to the external device.

Embodiments of the present disclosure may also include, the method of claim 8, further including a step of energy harvesting from external sources, such as through inductive coupling, to recharge or maintain the device's power level.

Embodiments of the present disclosure may also include a system for *in vivo* measurement of metabolites, including the implantable device of claim 1. Embodiments may also include an external device for receiving and processing the transmitted metabolite concentration and identity information. Embodiments may also include a user interface for displaying the metabolite concentration and identity information to a user.

Embodiments of the present disclosure may also include the system of claim 10. In some embodiments, the external device may include a data storage module for storing the received metabolite concentration and identity information for historical analysis or future reference.

Embodiments of the present disclosure may also include the system of claim 10. In some embodiments, the external device may include a data processing module for performing additional analysis on the received metabolite concentration and identity information, including comparison with reference data, trending, or statistical analysis.

Embodiments of the present disclosure may also include the system of claim 10. In some embodiments, the user interface may be configured to provide visual, auditory, or tactile feedback to the user based on the metabolite concentration and identity information.

Embodiments of the present disclosure may also include the system of claim 10. In some embodiments, the external device and the user interface may be integrated into a single unit, such as a smartphone, tablet, or dedicated monitoring device.

Embodiments of the present disclosure may also include the system of claim 10. In some embodiments, the external device may be configured to communicate with other devices, systems, or networks to share the metabolite concentration and identity information for remote monitoring, collaboration, or integration with electronic health records.

Embodiments of the present disclosure may also include, the system of claim 10, further including an alert module configured to generate alerts or notifications based on predetermined thresholds, patterns, or changes in the metabolite concentration and identity information.

Embodiments of the present disclosure may also include the system of claim 10. In some embodiments, the implantable device, the external device, and the user interface may be configured to receive software updates or configuration changes to improve the accuracy, sensitivity, or specificity of the metabolite measurements, or to add support for new metabolites.

Embodiments of the present disclosure may also include a method for monitoring metabolite concentrations and identities in a subject's tissue using the system of claim 10, including the steps of implanting the implantable device in the subject's body (human or another animal). Embodiments may also include measuring metabolite concentrations and identities *in vivo* using the implantable device, including generating Raman scattering signals, refining the signals with the Fabry-Pérot interferometer and the spherical mirror, capturing the signals using the CMOS sensor, and processing the signals to determine metabolite concentrations and identities.

Embodiments may also include transmitting the metabolite concentration and identity information from the implantable device to the external device using wireless communication protocols. Embodiments may also include processing and analyzing the received metabolite concentration and identity information on the external device, including comparison with reference data, trending, or statistical analysis. Embodiments may also include displaying the metabolite concentration and identity information on the user interface. Embodiments may also include generating alerts or notifications based on predetermined thresholds, patterns, or changes in the metabolite concentration and identity information using an alert module.

Embodiments of the present disclosure may also include, the method of claim 15, further including the step of storing the metabolite concentration and identity information in a data storage module on the external device for historical analysis or future reference.

Embodiments of the present disclosure may also include the method of claim 15. In some embodiments, the external device communicates with other devices, systems, or networks to share the metabolite concentration and identity information for remote monitoring, collaboration, or integration with electronic health records.

Embodiments of the present disclosure may also include, the method of claim 15, further including the step of receiving software updates or configuration changes on the implantable device, the external device, and the user interface to improve the accuracy, sensitivity, or specificity of the metabolite measurements, or to add support for new metabolites.

Embodiments of the present disclosure may also include, the method of claim 15, further including the step of customizing the user interface to display the metabolite concentration and identity information in a format that meets the needs or preferences of the user, including graphical representations, numerical values, or color-coded indicators.

Embodiments of the present disclosure may also include the method of claim 15. In some embodiments, the implantable device, the external device, and the user interface may be configured to perform self-diagnostic tests or calibration procedures to ensure the accuracy and reliability of the metabolite concentration and identity measurements.

Embodiments of the present disclosure may also include the method of claim 15. In some embodiments, the implantable device may be configured to adaptively adjust its sampling frequency or measurement parameters based on the subject's physiological state, activity level, or external factors to optimize the accuracy and relevance of the metabolite concentration and identity information.

Embodiments of the present disclosure may also include, the method of claim 15, further including the step of providing feedback or recommendations to the subject or healthcare provider based on the metabolite concentration and identity information, including dietary or lifestyle modifications, medication adjustments, or further diagnostic testing.

Embodiments of the present disclosure may also include the method of claim 15. In some embodiments, the implantable device may be configured to operate in a low-power mode or to temporarily suspend measurements during periods of inactivity, low energy availability, or to preserve the device's power supply.

Embodiments of the present disclosure may also include the method of claim 15. In some embodiments, the implantable device and the external device may be configured to synchronize their operation, data transmission, or other interactions to optimize power consumption, data accuracy, or user experience.

Embodiments of the present disclosure may also include, the method of claim 15, further including the step of integrating the metabolite concentration and identity information with other physiological data or measurements from the subject, such as heart rate, blood pressure, glucose levels, or oxygen saturation, to provide a comprehensive view of the subject's health status, to support decision-making by healthcare providers, or to enable personalized treatment plans.

Embodiments of the present disclosure may also include, the method of claim 15, further including the step of using machine learning, artificial intelligence, or pattern recognition techniques on the external device or a remote server to identify trends, correlations, or anomalies in the metabolite concentration and identity information, and to refine or optimize the analysis of the data.

Embodiments of the present disclosure may also include the method of claim 15. In some embodiments, the implantable device may be configured to be removed, replaced, or upgraded without causing significant discomfort or harm to the subject.

Embodiments of the present disclosure may also include, the method of claim 15, further including the step of maintaining the security and privacy of the subject's metabolite concentration and identity information through encryption, access control, or other data protection techniques during data transmission, storage, or processing.

Embodiments of the present disclosure may also include, the method of claim 15, further including the step of using the implantable device for monitoring metabolite concentrations and identities in animal subjects for research, drug development, or veterinary applications.

Embodiments of the present disclosure may also include the method of claim 15. In some embodiments, the implantable device, the external device, and the user interface may be configured to comply with applicable regulatory standards, guidelines, or requirements related to medical devices, patient safety, or data privacy.

Embodiments of the present disclosure may also include the method of claim 15. In some embodiments, the implantable device may be configured to perform continuous, semi-continuous, or periodic measurements of metabolite concentrations and identities, depending on the user's needs or preferences, or the specific clinical application.

Embodiments of the present disclosure may also include, the method of claim 15, further including the step of providing user support, troubleshooting, or remote diagnostics for the implantable device, the external device, or the user interface through a dedicated support channel, online platform, or customer service.

Embodiments of the present disclosure may also include the method of claim 15. In some embodiments, the implantable device may be configured to be used in combination with other medical devices or therapies, such as insulin pumps, continuous glucose monitors, or medication delivery systems, to optimize treatment outcomes or patient compliance.

Embodiments of the present disclosure may also include, the method of claim 15, further including the step of customizing the implantable device, the external device, or the user interface to accommodate the specific needs or preferences of different user populations, such as pediatric patients, elderly patients, or patients with specific medical conditions.

Embodiments of the present disclosure may also include the method of claim 15. In some embodiments, the implantable device may be configured to be used in various tissue types or anatomical locations within the subject's body (human or another animal), depending on the desired application or target metabolite.

Embodiments of the present disclosure may also include, the method of claim 15, further including the step of calibrating the implantable device using reference materials or known metabolite concentrations to ensure accurate and reliable measurements *in vivo.*

Embodiments of the present disclosure may also include the method of claim 15. In some embodiments, the implantable device may be configured to be used in a range of environmental conditions, such as varying temperatures, humidity levels, or pressures, while maintaining accurate and reliable metabolite concentration and identity measurements.

Embodiments of the present disclosure may also include, the method of claim 15, further including the step of providing training, education, or instructional materials to the subject, healthcare provider, or other users of the system to ensure proper implantation, operation, and interpretation of the metabolite concentration and identity information.

Embodiments of the present disclosure may also include the method of claim 15. In some embodiments, the implantable device may be configured to detect and measure a wide range of metabolites, including but not limited to, amino acids, lipids, carbohydrates, nucleic acids, vitamins, cofactors, or metabolic intermediates.

Embodiments of the present disclosure may also include the method of claim 15. In some embodiments, the implantable device may be configured to provide real-time, near-real-time, or delayed feedback on the metabolite concentration and identity information to enable timely interventions, adjustments, or decision-making by the subject or healthcare provider. In some embodiments, these claims cover various aspects of the implantable device, the associated system, and the method of using it for *in vivo* measurement of metabolites.

The advantage of the developed implantable metabolite monitoring system is the ability to process and analyze the received metabolite concentration and identification information on an external device, including comparison with reference data, trends or statistical analysis. Therefore, the methods allow generating alerts or notifications based on predetermined thresholds, patterns or changes in metabolite concentrations and metabolite detection information using the alert module.

The stage of storing metabolite concentration and detection information in the data storage module on an external device is used for historical analysis or future reference.

Receiving software updates or configuration changes on the implantable device, external device and user interface allows improving the accuracy, sensitivity or specificity of metabolite measurements or adding support for new metabolites.

Customization of the user interface allows the metabolite concentration and identity information to be displayed in a format that meets the user's needs or preferences. The interface can include graphical representations, numerical values or color-coded indicators.

The implantable device, external device and user interface can be configured to perform self-diagnostic tests or calibration procedures to ensure accuracy and reliability of measurements.

The implantable device can be configured to adaptively adjust sampling frequency or measurement parameters based on the user's physiological state, activity level or external factors to optimize the accuracy and relevance of metabolite concentration and identification information.

It is also possible to provide feedback or recommendations to a healthcare provider or subject based on metabolite concentrations and identification information, and thus dietary or lifestyle modifications, medication adjustments or further diagnostic testing.

The implantable device can be configured to operate in low-power mode or to temporarily suspend measurements during periods of inactivity, low energy availability or to conserve power to the implantable device.

The implantable device and an external device can be configured to synchronize their operation, data transmission or other interactions to optimize energy consumption, data accuracy or user experience.

The step of integrating metabolite concentration and identification information with other physiological data or measurements from the subject, such as heart rate, blood pressure, glucose levels or oxygen saturation, to provide a comprehensive picture of the subject's health, support decision-making by healthcare providers or enable personalized treatment plans.

The use of machine learning, artificial intelligence or pattern recognition techniques on an external device or remote server can identify trends, correlation or anomaly in metabolite concentrations and identification information, and to refine or optimize data analysis.

The implantable device can also be configured for removal, replacement or upgrade without causing significant discomfort or harm to the user.

It is possible to maintain the security and privacy of the metabolite concentration information and its identification of the subject through encryption, access control or other data protection techniques during data transmission, storage or processing. It is possible for the data contained in the device to be made available to medical services in the event of a threat to the user's life or health.

It is also possible to use an implantable device to monitor metabolite concentrations and identification in animals for research, drug development or veterinary applications.

The implantable device, external device and user interface can be configured to comply with applicable regulatory standards, guidelines or requirements related to medical devices, user safety or data privacy.

The implantable device can be configured to perform continuous, semi-continuous or periodic measurements of concentrations and metabolite identification, depending on the needs or preferences of the user or the specific clinical application.

The developed method also provides user support, troubleshooting or remote diagnostics of the implantable device, external device or user interface via a dedicated support channel, web platform or customer service.

The implantable device can be configured for use in conjunction with other medical devices or therapies, such as insulin pumps, continuous glucose monitors or drug delivery systems, to optimize treatment outcomes or user compliance.

It is also possible to customize the implantable device, external device or user interface to suit the specific needs or preferences of different user populations, such as children, the elderly or people with certain medical conditions.

The implantable device can be configured for use in different tissue types or anatomical locations in the user's body, depending on the desired application or target metabolite.

A calibration step of the implantable device using reference materials or known metabolite concentrations enables the device to provide accurate and reliable *in vivo* measurements.

The developed method also provides user support, troubleshooting or remote diagnostics of the implantable device, external device or user interface via a dedicated support channel, web platform or customer service.

The implantable device can be configured for use in conjunction with other medical devices or therapies, such as insulin pumps, continuous glucose monitors or drug delivery systems, to optimize treatment outcomes or user compliance.

It is also possible to customize the implantable device, external device or user interface to suit the specific needs or preferences of different user populations, such as children, the elderly or people with certain medical conditions. The implantable device can be configured for use in different tissue types or anatomical locations in the user's body, depending on the desired application or target metabolite. A calibration step of the implantable device using reference materials or known metabolite concentrations enables the device to provide accurate and reliable *in vivo* measurements.

It is also possible to customize the implantable device, external device or user interface to suit the specific needs or preferences of different user populations, such as children, the elderly or people with certain medical conditions.

The implantable device can be configured for use in different tissue types or anatomical locations in the user's body, depending on the desired application or target metabolite.

A calibration step of the implantable device using reference materials or known metabolite concentrations enables the device to provide accurate and reliable *in vivo* measurements.

The implantable device can be configured for use under a variety of external environmental conditions (which occur outside the user's body), such as different temperatures, humidity levels or pressures, while maintaining accurate and reliable measurements of metabolite concentration and identification.

The implantable device can be configured to provide real-time, near real-time or delayed feedback on metabolite concentrations and identification information to enable timely interventions, adjustments or decision-making by the user or healthcare professional.

### BRIEF DESCRIPTION OF THE FIGURES

The subject of the invention has been presented in the embodiments in which:
FIG. 1 is a block diagram illustrating an implantable device, according to some embodiments of the present disclosure.
FIG. 2 is a block diagram illustrating the implantable device, according to some embodiments of the present disclosure.
FIG. 3 is a block diagram illustrating the implantable device, according to some embodiments of the present disclosure showing the configuration order and the relationship of the various elements described in claims 1 and 3.
FIG. 4 is a block diagram illustrating the implantable device, according to some embodiments of the present disclosure.
FIG. 5 is a block diagram illustrating the implantable device, according to some embodiments of the present disclosure.
FIG. 6 is a flowchart illustrating a method, according to some embodiments of the present disclosure.
FIG. 7 is a flowchart illustrating a method for monitoring metabolite concentrations and identities in a subject's tissue, according to some embodiments of the present disclosure.

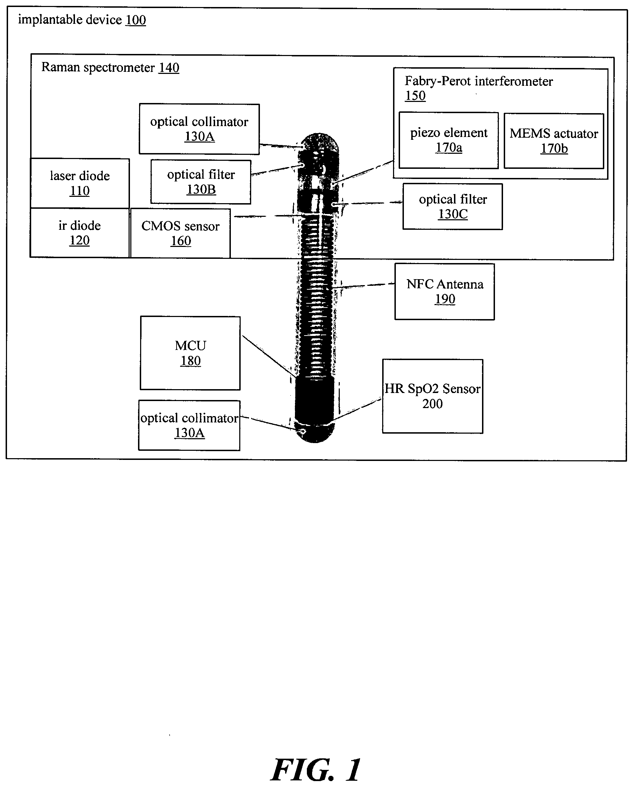

FIG. 1 is a block diagram that describes an implantable device 100, according to some embodiments of the present disclosure.

The implantable device 100 is implanted subcutaneously in the user's body depending on the metabolites to be tested, such as e.g. glucose, amino acids, lipids, carbohydrates, nucleic acids, vitamins, cofactors or metabolic intermediates, e.g. for liver enzyme testing the implantable device 100 will be placed close to the liver.

The implantation procedure of the implantable device 100 is performed by an injection performed with a known implantable syringe.

The implantable device 100 represents a device that is implanted anywhere under the skin into the inter-tissue space.

The implantable device 100 include a laser diode 110, an IR diode 120, an optical filter 130 that filtering the scattered light, a Raman spectroscopy unit 140 for detecting the metabolic profile of a subject, a CMOS sensor 160 for capturing the refined Raman scattering signals, a piezo element 170a or MEMS actuator 170b for actuating the Fabry-Pérot interferometer 150, and MCU 180 as a controller and a processing unit. The implantable device 100 may also include a Fabry-Pérot interferometer 150 with a spherical semi-mirror, functioning as an etalon, for refining the detection of Raman scattering signals.

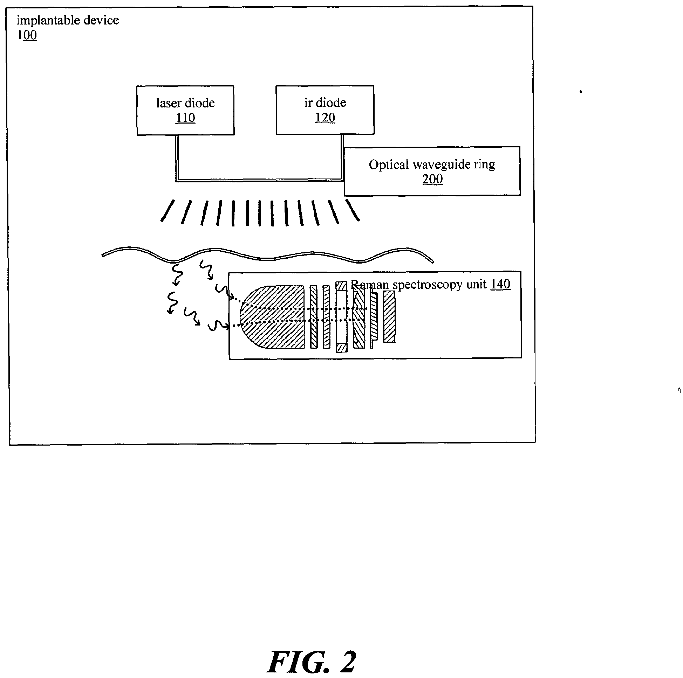

FIG. 2 is a block diagram that describes the implantable device 200, according to some embodiments of the present disclosure. In some embodiments, the Raman spectroscopy unit may be configured to used Raman scattering signals in response to incident light from laser diode 110 or IR diode 120 that is guided to subject body via optical waveguide ring 200, that light further interacting with the metabolites in the subject's tissue.

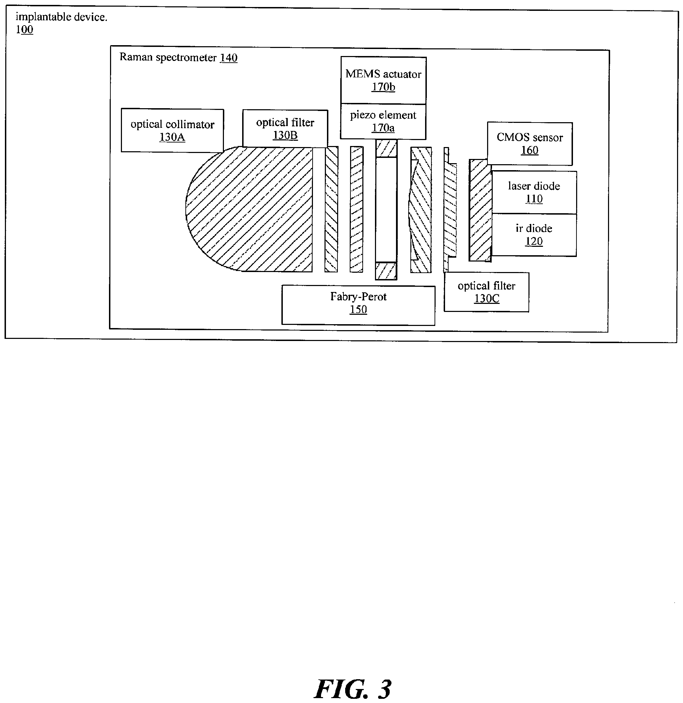

FIG. 3 is a block diagram that describes the implantable device 100, especially Raman spectrometer unit 140 according to some embodiments of the present disclosure. In some embodiments, the optical collimator 130a that collects light with a optical filter 130 that filtered out main wavelength from light source and the light is passed through the Fabry-Pérot interferometer 150 with a spherical semi-mirror with also piezo element 170a or MEMS actuator 170b and may be configured to selectively filtering specific wavelengths of the Raman scattering signals corresponding to the metabolites of interest.

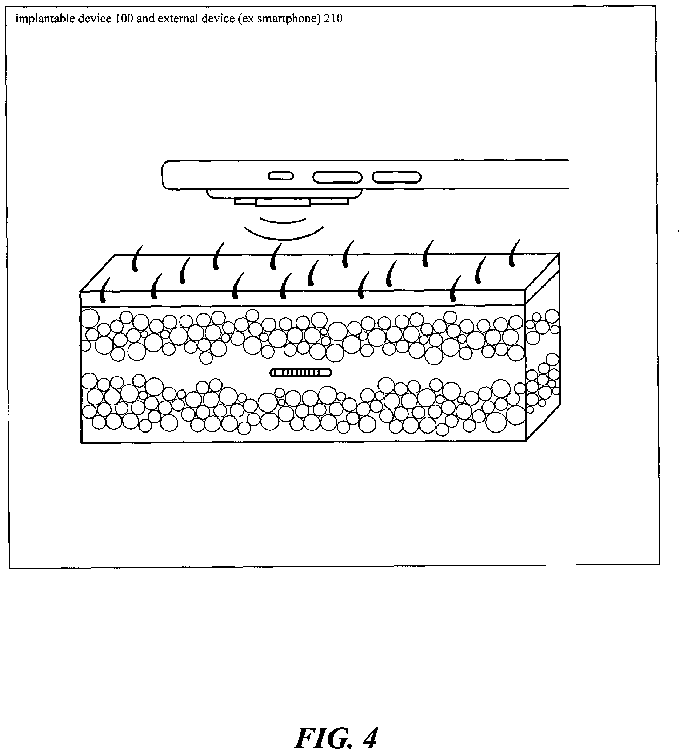

FIG. 4 is a block diagram that describes the implantable device 100, according to some embodiments of the present disclosure. In some embodiments, the external device (for example smartphone) 210 may be configured to power up through inductive coupling the implantable device 100, then via inductive coupling transmitting from implantable device 100 the Raman scattering signals represents of the metabolite concentrations in the subject's tissue (human or another animal).

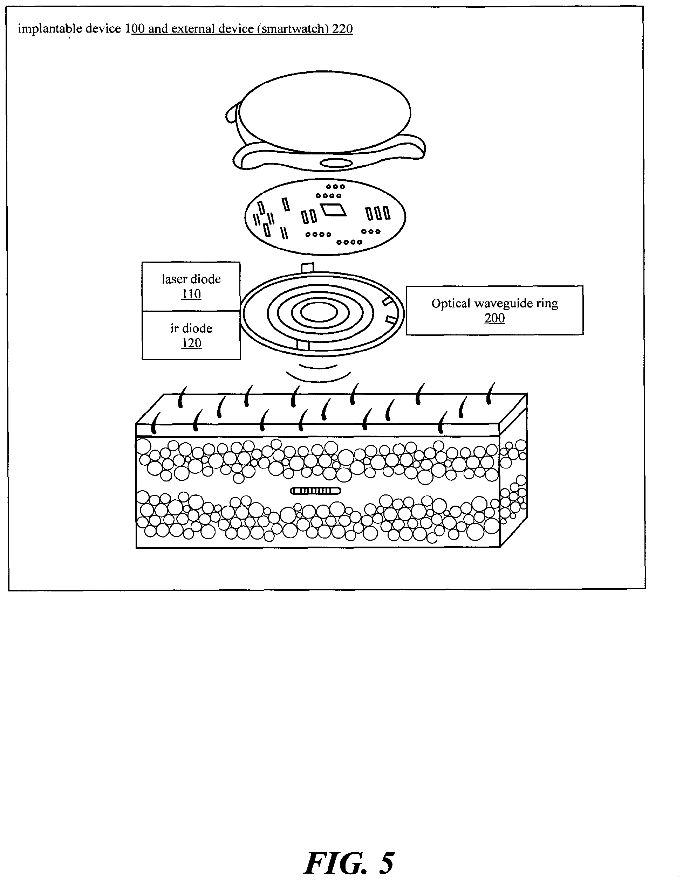

FIG. 5 is a block diagram that describes the implantable device 100, according to some embodiments of the present disclosure. In some embodiments, the external device 210 (for example smartwatch) may be configured to power up through inductive coupling the implantable device 100, then the external device emits light from laser diode 110 or IR diode and transmits through optical waveguide ring 200 and then via inductive coupling transmitting from implantable device 100 the Raman scattering signals represents of the metabolite concentrations in the subject's tissue (human or another animal).

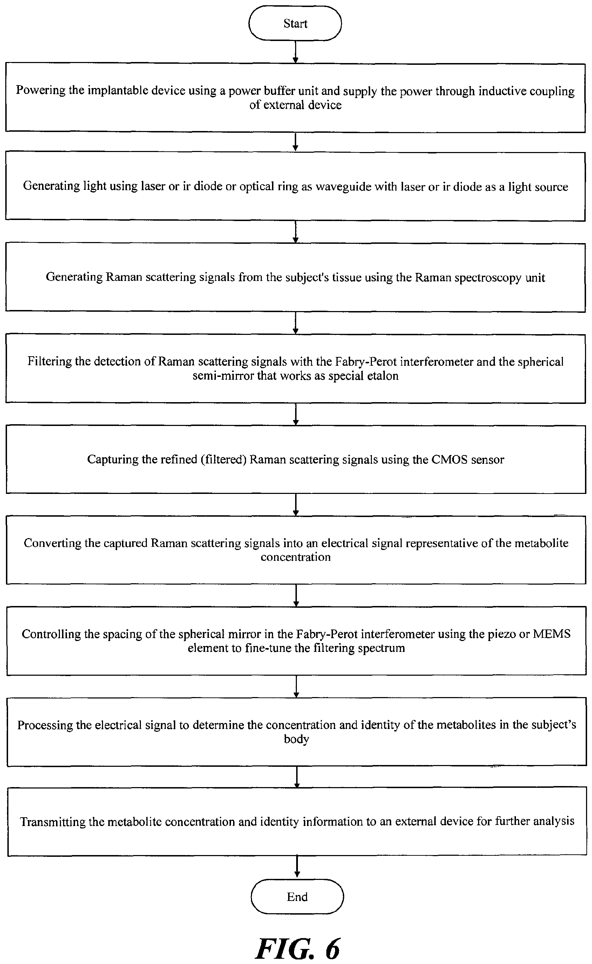

FIG. 6 is flowchart that describe a method, according to some embodiments of the present disclosure. In some embodiments, the method may include power up the implantable device 100 through inductive coupling of external device 210. In some embodiments, the method may include generating light using laser diode 110 or IR diode 120. The method may include generating Raman scattering signals from the subject's tissue using the Raman spectroscopy unit 140. The method may include refining the detection of Raman scattering signals with the Fabry-Pérot interferometer 150 and the spherical semi-transparent mirror. The method may include capturing the refined Raman scattering signals using the CMOS sensor 160. In some embodiments, the method may include converting the captured Raman scattering signals into an electrical signal representative of the metabolite concentrations in the subject's tissue using the CMOS sensor 160. The method may include controlling the spacing of the spherical mirror in the Fabry-Pérot interferometer 150 using the piezo element 170a or MEMS actuator 170b to fine-tune the detected Raman scattering signals. The method may include processing the electrical signal to determine the concentration and identity of the metabolites in the subject's tissue using a data processing unit. The method may include transmitting the metabolite concentration and identity information to an external device 210 for further analysis, display, or storage using a communication module.

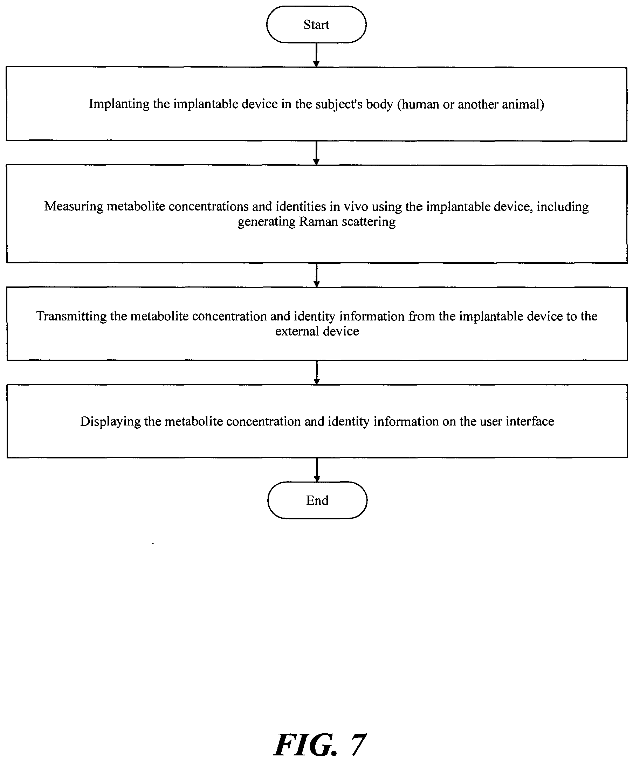

FIG. 7 is a flowchart that describes a method for monitoring metabolite concentrations and identities in a subject's tissue, according to some embodiments of the present disclosure. In some embodiments, the method may include implanting the implantable device 100 in the subject's body (human or another animal). The method may include measuring metabolite concentrations and identities *in vivo* using the implantable device 100, including generating Raman scattering signals, refining the signals with the Fabry-Pérot interferometer 150 and the spherical semi-transparent mirror, capturing the signals using the CMOS sensor 160, and processing the signals to determine metabolite concentrations and identities. The method may include transmitting the metabolite concentration and identity information from the implantable device 100 to the external device 210 using wireless communication protocols. The method may include displaying the metabolite concentration and identity information on the user interface.

### List of elements:

100. implantable device
110. laser diode
120. IR diode
130. optical filter
130a. optical collimator
140. Raman spectroscopy unit
150. Fabry-Pérot interferometer
160. CMOS sensor
170a. piezo element
170b. MEMS actuator
180. MCU
200. optical waveguide ring
210. external device

## Claims

1. An implantable device **(100)** for *in vivo* measurement of metabolites:
**characterized in that**
the implantable device (100) comprises,
a laser diode (110) or an IR diode (120),
an optical filter (130) that filters the scattered light,
a Raman spectroscopy unit (140) for detecting the metabolic profile of a subject,
a Fabry-Pérot interferometer (150) with a spherical mirror, functioning as an etalon, for refining the detection of Raman scattering signals,
a CMOS sensor (160) for capturing the refined Raman scattering signals,
a piezo element (170a) or MEMS actuator (170b) for actuating the Fabry-Pérot interferometer (150),
MCU (180) as a controller and a processing unit.

2. The implantable device (100) according to claim 1, **characterized in that** the laser diode (110) or the IR diode (120) is inside or outside as an optical waveguide ring (200) with laser diode (110) or IR diode (120) as a light source.

3. The implantable device (100) according to claim 1 or 2, **characterized in that** it includes the Fabry-Pérot interferometer (150) with a flat and spherical semi-transparent mirrors and the piezo element (170a) or MEMS actuator (170b) to adjust spacing between semi-transparent mirrors, refining the detection of specific metabolite-related Raman scattering light wavelengths.

4. The implantable device (100) according to claim 3, **characterized in that** the semi-transparent mirrors are created from optical fiber bundle or optical fiber plate or homogenous glass.

5. The implantable device (100) according to claim 1 or 2 or 3 or 4, **characterized in that** the MCU (180) as the data processing unit within the device analyzes interference fringes from Fabry-Pérot interferometer (150) as the CMOS sensor (160) generated electrical signal to determine metabolite identity and concentration, employing machine learning or pattern recognition techniques aligned with known Raman spectra.

6. The implantable device (100) according to claim 1 or 2 or 3 or 4 or 5, **characterized in that** the implantable device (100) encompasses a communication module, preferably with wireless capabilities, to transmit metabolite data to external devices for further processing, display, or storage.

7. The implantable device (100) according to claim 1 or 2 or 3 or 4 or 5 or 6, **characterized in that** the implantable device (100) is biocompatible for long-term implantation, supported by a power buffer unit, which can be recharged or sustained via energy harvested from external sources like inductive coupling.

8. A method for *in vivo* measurement of metabolites using the implantable device (100) of claim 1, comprising the steps of:
a. generating light using laser diode (110) or IR diode (120) or optical waveguide ring (200) with laser diode (110) or IR diode (120),
b. generating Raman scattering signals from the subject's tissue using the Raman spectroscopy unit (140),
c. refining the detection of Raman scattering signals with the Fabry-Pérot interferometer (150) and flat and spherical semi-transparent mirrors,
d. capturing the refined Raman scattering signals using the CMOS sensor (160),
e. converting the captured Raman scattering signals into an electrical signal representative of the metabolite concentrations in the subject's tissue using the CMOS sensor (160),
f. controlling the spacing between flat and spherical semi-transparent mirrors in the Fabry-Pérot interferometer (150) using the piezo element (170a) or MEMS actuator (170b) to fine-tune the detected Raman scattering signals,
g. processing the electrical signal to determine the concentration and identity of the metabolites in the subject's tissue using a MCU (180) as a data processing unit,
h. transmitting the metabolite concentration and identity information to an external device for further analysis, display, or storage using a communication module and
i. powering the implantable device using a power buffer unit and supplying the power through inductive coupling of external device.

9. The method involving the implantable device (100) according to **claim 8, characterized in that** the method includes steps for generating and refining Raman signals, converting them to an electrical representation, processing this data to discern metabolite details, and transmitting this information externally, all powered *via* the short-range wireless connectivity.

10. A system for *in vivo* measurement of metabolites, **characterized in that** the system comprising:
a. the implantable device (100),
b. an external device (210) for receiving and processing the transmitted metabolite concentration and identity information,
c. a user interface for displaying the metabolite concentration and identity information to a user.

11. The system according to claim 10, **characterized in that** it receives updates to enhance performance, interface with other devices or health records, and generate alerts based on specific metabolite information parameters or thresholds.

12. The system according to claim 10 or 11, **characterized in that** the implantable device (100) is placed within a subject to measure metabolite concentrations and identities *in vivo* using Raman scattering signals and this process includes refining signals with specific equipment and processing these signals to identify the metabolites.

13. The system according to claim 10 or 11 or 12, **characterized in that** the system uses advanced techniques, like machine learning, refines the data analysis and users can access support channels for troubleshooting, and training is provided to ensure proper system usage.

14. The system according to claim 10 or 11 or 12 or 13, **characterized in that** the system is used in diverse applications, including research and various clinical settings.

15. A method for monitoring metabolite concentrations and identities in a subject's tissue using the system of claim 10, comprising the steps of:
a. implanting the implantable device (100) in the human or another animal body,
b. measuring metabolite concentrations and identifying *in vivo* using the implantable device (100), including generating Raman scattering signals, refining the signals with the Fabry-Pérot interferometer (150) and the flat and spherical semi-transparent mirrors, capturing the signals using the CMOS sensor (160), and processing the signals to determine metabolite concentrations and identities,
c. transmitting the metabolite concentration and identity information from the implantable device (100) to the external device (210) using wireless communication protocols,
d. processing and analyzing the received metabolite concentration and identity information on the external device (210), including comparison with reference data trending or statistical analysis,
e. displaying the metabolite concentration and identity information on the user interface and
f. generating alerts or notifications based on predetermined thresholds, patterns, or changes in the metabolite concentration and identity information using an alert module.

16. The method according to claim 15, **characterized in that** the implantable device (100) wirelessly transmits the data to an external device (210), where it is processed, stored for future reference, and analyzed and this includes comparison with other data and the potential for historical analysis, security and privacy mechanisms protect this data.

17. The method according to claim 15 or 16, **characterized in that** the external device (210) provides a customizable user interface to display the metabolite data, generating alerts based on specific criteria and users receive updates or configuration changes for the system, and there are options for synchronization and integration with other devices or health records.

18. The method according to claim 15 or 16 or 17, **characterized in that** the implantable device (100) adapts its measurements based on various factors related to the subject, it offers feedback to the subject or healthcare providers, including potential recommendations based on the data.

19. The method according to claim 15 or 16 or 17 or 18, **characterized in that** advanced techniques, like machine learning, refine the data analysis, users access support channels for troubleshooting, and training is provided to ensure proper system usage, the system is used in diverse applications, including research and various clinical settings.
